# EUROPÄISCHE PATENTANMELDUNG

(11) **EP 0 782 871 A2**
(43) Veröffentlichungstag der Anmeldung: **09.07.1997**
(21) Anmeldenummer: 96117041.2
(22) Anmeldetag: 24.10.1996
(51) Int. Cl.: A61N 5/06

(54) **Verfahren und Strahlungsanordnung zur Erzeugung von UV-Strahlen zur Körperbestrahlung sowie Verwendung**

(30) Priorität: 22.11.1995 DE 19543342
(71) Anmelder: Heraeus Noblelight GmbH, 63450 Hanau (DE)
(72) Erfinder: Arnold, Erich, Dr., 55122 Mainz (DE); Maul, Friedel, 63526 Erlensee (DE); Dohn, Alexander, Dr., 63694 Limeshain-Hainchen (DE)
(74) Vertreter: Kühn, Hans-Christian

(57) **Zusammenfassung**

Zur Körperbestrahlung mit UV-Strahlen wird eine inkohärente Excimierstrahlung im Wellenlängenbereich im Bereich von 300 bis 315 nm - vorzugsweise 308 nm - erzeugt, wobei in einer Koaxial-Anordnung ein hohlzylindrisches transparentes Dielektrikum vorgesehen ist, das auf seiner Außenoberfläche eine strahlungsdurchlässige netzförmige Metallelektrode aufweist und im Inneren eine entlang der Zylinderachse verlaufende stabförmige Innenelektrode aus Wolfram enthält; der von dem ringmantelförmigen Dielektrikum umschlossene Entladungsraum enthält eine Xenonhalogenid-Füllung, vorzugsweise eine Xenonchlorid-Füllung mit einem Kaltfülldruck von 500 bis 1500 mbar; die zwischen der inneren und äußeren Elektrode anliegende Wechselspannung kann durch Modulation mittels Tastverhältnis dem jeweiligen Zweck der Körperbestrahlung z.B. Bräunung angepaßt werden.

## Beschreibung

Die Erfindung betrifft ein Verfahren zur Erzeugung von UV-Strahlen zur Körperbestrahlung sowie eine Strahlungsanordnung zur Körperbestrahlung sowie die Verwendung des Verfahrens, bzw. der Strahlungsanordnung.

Aus der DE 43 24 007 A1 ist ein Verfahren für eine hautschonende Emission bekannt, bei der für den Einsatz in Bräunungsanlagen eine Lichtquelle eingesetzt ist, die keine oder reduzierte Anteile von langwelliger UV-A-Strahlung aufweist; als Lichtquelle wird dabei eine Strahlerfilterkombination eingesetzt, wobei es sich bei den Strahlern um eine undotierte Hg-Hochdrucklampe handelt.

Als problematisch erweist es sich, daß aufgrund von Filterabsorption ein Teil der Strahlungsleistung als Verlustwärme verloren geht, wobei erhöhte Wärme in der Strahlerfilterkombination entstehen kann.

Weiterhin ist aus der EP 0 254 111 B1 ein UV-Strahler bekannt, der einen mit Füllgas gefüllten, von Wänden begrenzten Entladungsraum enthält, wobei mindestens eine Wand von einem Dielektrikum gebildet wird und wobei zwei Elektroden vorgesehen sind, von denen wenigstens eine erste Elektrode auf der dem Entladungsraum abgewandten Oberfläche des Dielektrikums angeordnet ist; diese erste Elektrode besteht aus linien- oder streifenförmigem Metall, so daß sowohl die erste Elektrode als auch das Dielektrikum für die durch eine stille elektrische Entladung erzeugte Strahlung durchlässig sind, während die zweite Elektrode eine UV-strahlungsreflektierende Schicht, vorzugsweise Aluminiumschicht aufweist. Das Füllgas ist ein unter Entladungsbedingungen Excimere bildendes Edelgas oder Edelgasgemisch, wobei das Füllgas zusätzlich Quecksilber, Stickstoffselen, Deuterium oder ein Gemisch dieser Substanzen für sich oder allein mit einem Edelgas aufweisen kann; als problematisch erweist sich hierbei die verhältnismäßig kurzwellige Strahlung der Edelgase Helium, Neon, Argon, Xenon unter Excimierbildung im Bereich von 60 bis 190 nm, so daß dieser außerordentlich kurzwellige UV-Bereich des Spektrums für Körperbestrahlung bzw. für Bräunung nicht in Frage kommt; weiterhin ist eine Füllgasmischung aus Xenon und Chlor angegeben, die eine Strahlung im Bereich von 300 bis 320 nm, d.h. in dem für die Körperbestrahlung passenden Bereich angibt.

Aufgabe der Erfindung ist es, ausgehend von der EP 0 254 111 B1 mit einer Xenon und ein Halogen, insbesondere Chlor, enthaltenden Füllgasmischung ein Verfahren und eine Anordnung anzugeben, mit der eine intensive Körperbestrahlung im UV-A/UV-B-Bereich möglich ist; dabei soll eine monochromatische erythemwirksame Bestrahlung für die Langzeitpigmentierung erzeugt werden und eine gezielte Phototherapie hoher Intensität ohne nachteilige Überbeanspruchung der Haut ermöglicht werden; weiterhin soll eine Verwendung angegeben werden.

Die Aufgabe wird verfahrensgemäß durch das kennzeichnende Merkmal des Anspruchs 1 bzw. des Anspruchs 3 gelöst.

Als besonders vorteilhaft erweist es sich, daß hier keinerlei Filterstrahlerkombinationen mehr erforderlich sind und somit die zur Verfügung stehende Energie der Excimier-Strahlung weitestgehend ausgenutzt werden kann; dabei ist es auch möglich, eine dem jeweiligen Bedarf angepaßte Dosierung der Strahlung vorzunehmen, woraus sich eine Vielzahl von Verwendungsfällen ergibt.

Eine weitere vorteilhafte Ausgestaltung des Verfahrens ist in Anspruch 2 angegeben.

Die Aufgabe wird anordnungsgemäß durch eine Strahlungsanordnung gelöst, wie sie im kennzeichnenden Teil des Anspruchs 4 angegeben ist.

Als besonders vorteilhaft erweist es sich, daß die Strahlungsanordnung den unterschiedlichen Aufgabenstellungen der zu bestrahlenden Körpergeometrie angepaßt werden kann, da die Strahlungsanordnung beispielsweise in langgestreckter Form zur Gesamtkörperbestrahlung ausgebildet sein kann, jedoch auch durch Ausgestaltung von zu- bzw. abschaltbaren Teilelektroden eine Anpassung an die jeweils zu bestrahlende Körperpartie ermöglicht; darüberhinaus erweist es sich als besonders vorteilhaft, daß keinerlei Zündverzögerungen entstehen, so daß beispielsweise durch Einstellung eines bestimmten Tastverhältnisses (Impulsdauer zu Impulspause) d.h. durch Modulation der anliegenden Wechselspannung eine Optimierung der Bestrahlung auf den jeweiligen Anwendungszweck möglich ist; darüberhinaus erweist sich die hohe Standzeit der Strahlungsanordnung von weit über 1000 Stunden als besonders vorteilhaft.

Weitere vorteilhafte Ausgestaltungen der Strahlungsanordnung sind in den Ansprüchen 5 bis 11 angegeben.

Als besonders vorteilhaft erweist sich aufgrund der koaxialen Ausgestaltung des Strahlers die Möglichkeit, Strahler mit einem gegenüber herkömmlichen Strahlungsanordnungen stark verringerten Außendurchmesser einzusetzen, wobei sich die Segmentierung der äußeren ersten Elektrode zur genauen Anpassung an die Bedürfnisse der Bestrahlung, insbesondere bei Bräunung als besonders vorteilhaft erweist.

Die Aufgabe wird verwendungsgemäß durch die Ansprüche 12 und 13 gelöst.

Ein wesentlicher Vorteil ist weiterhin darin zu sehen, daß durch gezielte Einstellung von UV-A-zu UV-B-Anteil ein Ersatz der für diese Strahlung üblicherweise verwendeten verhältnismäßig kurzlebigen Quecksilberdampf-Niederdruckstrahler möglich ist.

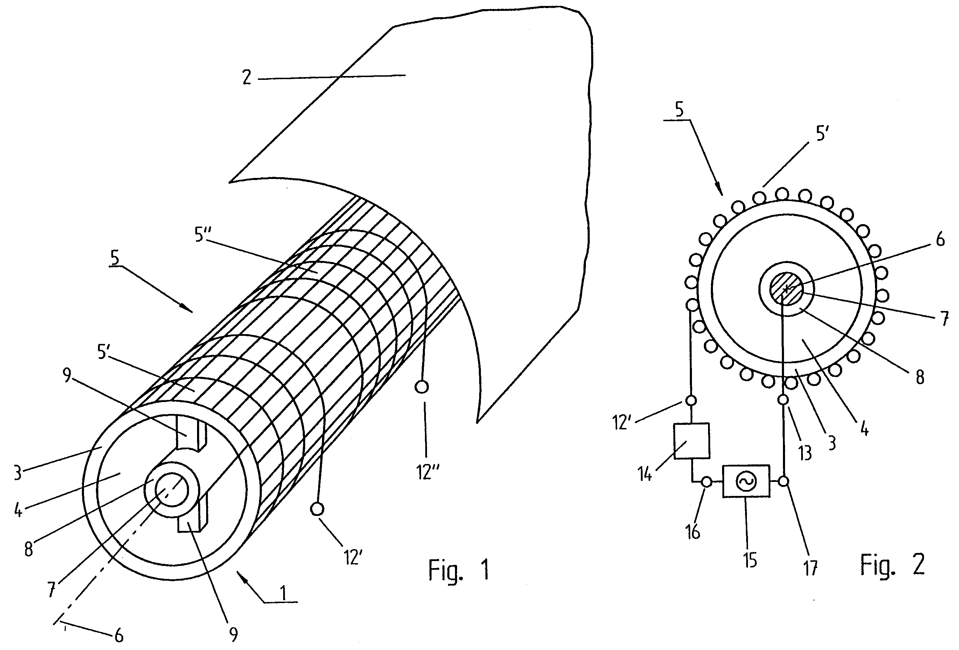

Im folgenden ist der Gegenstand der Erfindung anhand der Figuren 1 und 2 näher erläutert.
Figur 1 zeigt in einer perspektivischen Zeichnung eine teilweise aufgebrochen dargestellte Strahleranordnung;
Figur 2 zeigt einen Querschnitt durch den Strahler gemäß Figur 1.

Die Strahlungsanordnung nach Figur 1 besteht im wesentlichen aus dem aufgebrochen dargestellten Excimerstrahler 1 und einer ebenfalls bruchstückhaft dargestellten Reflektoranordnung 2. Der Excimierstrahler 1 weist einen von einem hohlzylindrisch ausgebildeten Dielektrikum 3 umgebenenen Entladungsraum 4 auf. Das Dielektrikum 3 ist für die im Entladungsraum erzeugte Strahlung transparent, wobei eine auf der Außenoberfläche des Dielektrikums 3 aufgebrachte erste Elektrode 5 aufgrund ihrer netzartigen Struktur ebenfalls für die im Entladungsraum erzeugte Strahlung durchlässig ist.

Im Inneren des Dielektrikums 3 ist entlang der Zylinderachse 6 eine stabförmige zweite Elektrode 7 angeordnet, die von einem zweiten Dielektrikum 8 umgeben ist. Die Halterung der zweiten Elektrode erfolgt durch Stützelemente.

Insbesondere bei langgestreckten Excimierstrahlern hat es sich als zweckmäßig erwiesen, voneinander im Abstand angeordnete Distanzelemente 9 aus elektrisch isolierendem Material vorzusehen, welche zwischen dem hohlzylindrisch ausgebildeten Dielektrikum 3 und dem zweiten Dielektrikum 8 angeordnet sind und die zweite Elektrode 7 zusammen mit Dielektrikum 8 mechanisch fest arretieren.

Die erste Elektrode 5 ist in mehrere ringförmige Segmente 5', 5'' aufgeteilt, um eine räumliche Anpassung der bestrahlten Fläche an die Körpergeometrie vornehmen zu können. Es ist dabei möglich, die Elektrodenanschlüsse 12', 12'' über hier nicht dargestellte Schalter bzw. steuerbare Schalter elektrisch miteinander zu verbinden bzw. elektrisch voneinander zu isolieren, so daß mehrere Ringsegmente als eine Elektrode 5 funktionieren bzw. nur ein Ringsegment - z.B. Ringsegment 5' - oder eine Auswahl von Ringsegmenten über den Schalter bzw. steuerbaren Schalter mit der Spannungsversorgung zur Erzeugung von UV-Strahlung verbunden sind.

Entladungsraum 4 ist an seinen beiden hier nicht dargestellten Stirnflächen in üblicher Weise geschlossen, wobei er vor der Einbringung des Füllgases evakuiert und dann mit dem bei Entladungsbedingungen Excimere bildenden Füllgas, im vorliegenden Fall mit Xenonhalogenid mit einem Kaltfülldruck im Bereich von 500 bis 1500 mbar gefüllt und hermetisch abgedichtet wird.

Die erste lichtdurchlässige Elektrode 5 besteht aus einem Netz aus VA-Stahl; beide Dielektrika 3 und 8 bestehen aus Quarz, wobei insbesondere das hohlzylindrisch ausgebildete Dielektrikum 3 aus Quarzglas besteht, um eine gute Transparenz für UV-Strahlung zu gewährleisten; die zweite, innenliegende Elektrode 7 besteht aus einem elektrisch leitenden Metallstab, vorzugsweise aus Wolfram, um eine hohe Temperaturbeständigkeit zu gewährleisten.

Die teilweise dargestellte Reflektoranordnung 2 besteht vorzugsweise aus Aluminium.

Anhand Figur 2 ist der Querschnitt durch das hohlzylindrische Dielektrikum 3 erkennbar, wobei entlang der Außenoberfläche die parallel zur Längsachse verlaufenden Teile des Ringsegments 5' der netzförmigen Elektrode 5 erkennbar sind; über einen hier schematisch dargestellten steuerbaren Schalter 14 können zwecks Parallel-Schaltung mehrere Ringsegmente als Teilelektroden der ersten Elektrode 5 miteinander verbunden werden, wobei der steuerbare Schalter 14 an Klemme 16 der Spannungsversorgung 15 als Wechselspannungsquelle angeschlossen ist.

Die zweite Elektrode 7 ist über Elektrodenanschluß 13 und Anschlußklemme 17 mit der Spannungsversorgung 15 verbunden. Anhand Figur 2 ist erkennbar, daß die zweite Elektrode 7 stabförmig entlang der Achse 6 geführt ist und von dem zweiten Dielektrikum 8 aus Quarz bzw. Quarzglas ringmantelförmig umgeben ist.

Entladungsraum 4 enthält eine Füllung aus Xenonchlorid, wobei zur Erzeugung von inkohärenter Excimierstrahlung mit einer Wellenlänge im Bereich von 300 bis 315 nm ein Kaltfülldruck im Bereich von 500 bis 1000 mbar vorgesehen ist; um eine Wellenlänge im Bereich von 308 nm zu erzielen, weist die Xenonchlorid-Füllung einen Kaltfülldruck von ca. 750 mbar auf.

Um eine inkohärente Excimierstrahlung mit einer Wellenlänge im Bereich von 315 bis 350 nm zu erzielen, weist der Entladungsraum 4 eine Xenonchlorid-Füllung mit einem Kaltfülldruck im Bereich von 750 bis 1500 mbar auf.

## Patentansprüche

1. Verfahren zur Erzeugung von UV-Strahlen zur Körperbestrahlung, dadurch gekennzeichnet, daß die Strahlen als inkohärente Excimerstrahlung im Wellenlängenbereich von 300 bis 315 nm emittiert werden.

2. Verfahren nach Anspruch 1, dadurch gekennzeichnet, daß die Strahlen mit einer Wellenlänge von 308 nm erzeugt werden.

3. Verfahren zur Erzeugung von UV-Strahlen zur Körperbestrahlung, dadurch gekennzeichnet, daß die Strahlen als inkohärente Excimerstrahlung im Wellenlängenbereich von 315 bis 350 nm emittiert werden.

4. Strahlungsanordnung zur Erzeugung von UV-Strahlen zur Körperbestrahlung, dadurch gekennzeichnet, daß ein mit unter Entladungsbedingungen Excimere bildenden Füllgas gefüllter Entladungsraum (4) vorgesehen ist, dessen eine Wand von einem Dielektrikum (3) gebildet ist, welches auf seiner dem Entladungsraum abgewandten Oberfläche mit einer ersten Elektrode (5) versehen ist, wobei zumindest diese Elektrode (5) und das Dielektrikum (3) strahlungsdurchlässig sind, wobei eine zweite Elektrode (7) vorgesehen ist, die den Entladungsraum (4) unmittelbar oder mittelbar begrenzt, und beide Elektroden (5, 7) an eine Spannungsquelle (15) angeschlossen sind, wobei der Entladungsraum eine Füllung aus Xenonhalogenid mit einem Kaltfülldruck von 500 bis 1500 mbar enthält.

5. Strahlungsanordnung nach Anspruch 4, dadurch gekennzeichnet, daß die zweite Elektrode (7) auf ihrer zur ersten Elektrode (5) gerichteten Oberfläche mit einem Dielektrikum (8) abgedeckt ist.

6. Strahlungsanordnung nach Anspruch 4 oder 5, dadurch gekennzeichnet, daß beide Elektroden (5, 7) zueinander koaxial ausgerichtet sind, wobei die auf dem hohlzylindrisch ausgebildeten Dielektrikum (3) aufliegende erste Elektrode (5) den Entladungsraum (4) in Form eines Ringmantels umgibt und die zweite Elektrode (7) sich entlang der Zylinderachse (6) des Ringmantels erstreckt, wobei sich entlang der Achse (6) gegenüberliegende Stirnwände den Ringmantel zwecks Bildung des Entladungsraums (4) hermetisch dicht abschließen.

7. Strahlungsanordnung nach Anspruch 6, dadurch gekennzeichnet, daß die erste Elektrode (5) aus wenigstens einem ringförmigen Segment (5', 5'') eines Metallnetzes besteht.

8. Strahlungsanordnung nach Anspruch 7, dadurch gekennzeichnet, daß die erste Elektrode (3) aus wenigstens zwei ringförmigen Segmenten (5', 5'') besteht, die über einen steuerbaren Schalter (14) miteinander elektrisch zu verbinden sind.

9. Strahlungsanordnung nach einem der Ansprüche 4 bis 8, dadurch gekennzeichnet, daß der Entladungsraum (4) zur Erzeugung von inkohärenter Excimer-Strahlung mit einer Wellenlänge im Bereich von 300 bis 315 nm Xenonchlorid mit einem Kaltfülldruck im Bereich von 500 bis 1000 mbar enthält.

10. Strahlungsanordnung nach Anspruch 9, dadurch gekennzeichnet, daß der Entladungsraum (4) zur Erzeugung einer Excimerstrahlung mit einer Wellenlänge von 308 nm Xenonchlorid mit einem Kaltfülldruck von ca. 750 mbar enthält.

11. Strahlungsanordnung nach einem der Ansprüche 4 bis 8, dadurch gekennzeichnet, daß der Entladungsraum (4) zur Erzeugung einer inkohärenten Excimerstrahlung mit einer Wellenlänge im Bereich von 315 bis 350 nm Xenonchlorid mit einem Kaltfülldruck im Bereich von 750 bis 1500 mbar enthält.

12. Verwendung des Verfahrens nach einem der Ansprüche 1 bis 3 zur Phototherapie oder zur Bräunung des menschlichen Körpers.

13. Verwendung der Strahlungsanordnung nach einem der Ansprüche 4 bis 11 zur Phototherapie oder zur Bräunung des menschlichen Körpers.
